# EUROPEAN PATENT SPECIFICATION

(11) **EP 2 110 159 B1**
(45) Date of publication and mention of the grant of the patent: **09.01.2013**
(21) Application number: 09005042.8
(22) Date of filing: 06.04.2009
(51) Int. Cl.: A61N 5/06, A61N 5/067

(54) **Device for irradiating the scalp with light sources**
Vorrichtung zur Bestrahlung der Kopfhaut mit Lichtquellen
Dispositif d'irradiation du cuir chevelu avec des sources lumineuses

(30) Priority: 15.04.2008 IT BG20080024
(43) Date of publication of application: 21.10.2009
(73) Proprietor: Dermotricos S.R.L., 25030 Coccaglio (BS) (IT)
(72) Inventor: Angoli, Roberto, 25032 Chiari (BS) (IT)
(74) Representative: Gatti, Enrico

(56) References cited:
- WO-A-03/039478
- WO-A-2004/026400
- WO-A1-2006/034033
- GB-A- 828 827
- GB-A- 2 293 770
- US-A1- 2006 161 226

## Description

The present invention relates to a device for irradiating the scalp with light sources, and to a relative method.

Scalp therapy by light sources, preferably laser, has recently provoked considerable interest. This therapy consists of irradiating the scalp with sources of laser light. Laser energy has shown to be able to increase the blood flow and circulation of the scalp.

Brushes with embedded laser light sources are commercially available for this purpose.

Document WO 2004/026400 discloses a headphone apparatus for hair growing comprising a frame for coupling left and right ear pads together and irradiating the head skin with a light emitted from a plurality of light emitting diodes arranged along the frame.

The object of the present invention is to provide a device for irradiating the scalp with light sources which is simple to use and enables the light radiation to be uniformly distributed over the scalp.

This and other objects are attained according to the present invention by a device according to claim 1.

Further characteristics of the invention are described in the dependent claims.

The treatment consists of irradiating the scalp with solid state light sources which emit laser light or non-laser light. The light wavelength can comprise all visible wavelengths including infrared and ultraviolet. The light beams can be focused or non-focused. The modality of emission, i.e. continuous or pulsed, and the treatment time can vary and are regulated by a microprocessor.

The apparatus is worn as a pair of spectacles and is provided with a movable structure which rotates about two fulcrums positioned above the ears. The movable structure incorporates the light emitters and is positionable both horizontally and vertically in order to adapt to the shape of the head. By rotating the movable structure the entire head vertex region, which is that mostly concerned with scalp pathologies or dysfunctions, can be progressively irradiated.

Rotation of the movable structure can be manual (in steps) or motorized with continuous movement by micro-motors inserted into the articulated joints.

The characteristics and advantages of the present invention will be apparent from the ensuing description of a practical embodiment thereof, illustrated by way of no-limiting example in the accompanying drawings, in which:

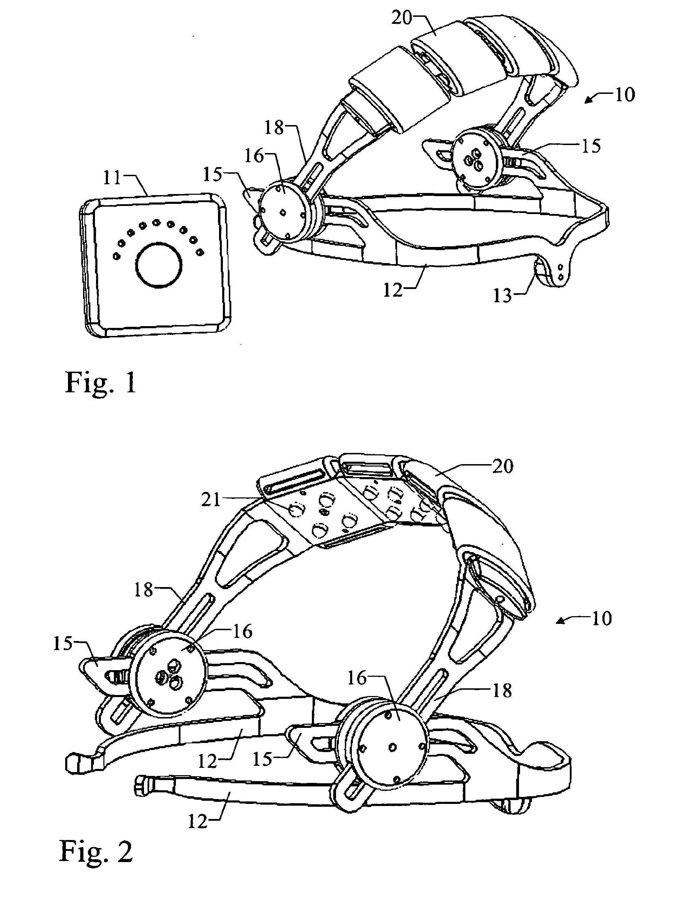

Figure 1 is a perspective view of a device for irradiating the scalp with light sources together with a control device, according to the present invention;
Figure 2 is a different perspective view of a device for irradiating the scalp with light sources, according to the present invention;

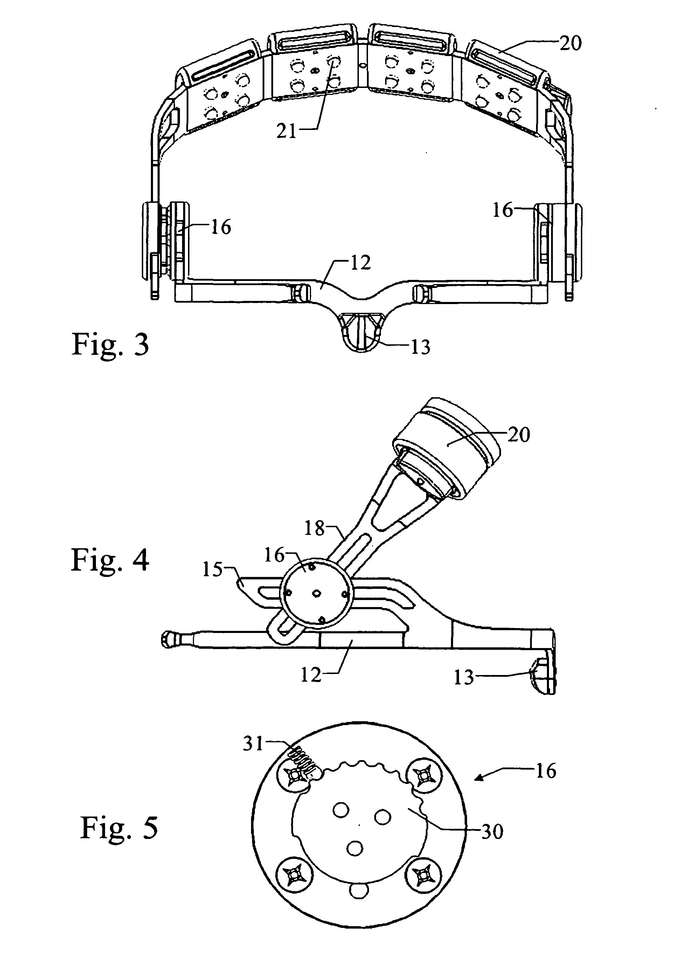

Figure 3 is a rear view of a device for irradiating the scalp with light sources, according to the present invention;
Figure 4 is a side view of a device for irradiating the scalp with light sources, according to the present invention;
Figure 5 shows an internal detail of a device for irradiating the scalp with light sources, according to the present invention.

With reference to the accompanying figures, a device for irradiating the scalp with light sources comprises an apparatus 10 to be worn as a pair of spectacles and a control and powering device 11, these being connected together by electrical connections, not shown.

The apparatus 10 has as its basis a crown arrangement 12 open to the rear. It can be closed rearwards by an elastic strap; or Velcro, to facilitate its positioning and maintenance.

A projection 13 is provided at its front extending towards the inside of the crown arrangement 13, for resting the apparatus against the user's forehead. The projection is made of soft material.

Two horizontally positioned forks 15, closed at their ends, are mounted above the side of the crown arrangement 12.

Two mechanisms or articulated joints 16 are mounted on these forks, one per side.

Two further forks 18, also closed at their ends, are inserted into these latter. The forks 18 are connected to the support structure 20 for the light emitters 21.

The structure 20, of semicircular shape, comprises a plurality of light emitters 21, of which 16 are provided in the illustrated example.

The structure 20 connected to the forks 18 is movable about the articulated joints 16.

The joints 16 are free to slide, under friction, along the forks 15 such as to move the structure 20 forwards or rearwards relative to the head.

The forks 18 are free to slide, under friction, through the joints 16 such as to move the structure 20 towards or away from the head.

The forks 18 can also rotate stepwise about the joints 16 by virtue of a toothed wheel 30 and a sprung pin 31, positioned within the joints.

The apparatus 10 is made of a plastic material which is elastic to enable it to adapt to the head.

After inserting the batteries into the control and powering device 11 for the apparatus 10, treatment can commence.

The apparatus 10 is worn in the manner of spectacles and the two articulated joints are adjusted horizontally and vertically such that the emitters are spaced from the head by 2 to 4 centimetres in all the nine possible positions.

The emitters are initially set in the first position (either front or rear).

The central pushbutton of the control device 11 is pressed until the device switches on, then the initial test step is awaited, indicated by the progressive lighting and extinguishing of all the indicator lights, accompanied by an acoustic signal of ascending tone.

On termination of the test step, the control device is ready to commence treatment, and awaits a command by the user. Under these conditions the indicator light relative to the initial position flashes rapidly.

If on termination of the testing step the batteries are depleted to the extent of not ensuring the execution of a complete treatment cycle, the device indicates this condition by repeated flashing of all indicator lights and the device automatically switches off while emitting an acoustic signal of descending tone.

To commence treatment, the treatment start button is quickly pressed. During treatment, the specific indicator light flashes slowly. On termination of treatment a short acoustic signal is emitted, the specific indicator light lights continuously, and the indicator light of the next position flashes rapidly.

The operation is repeated until the final position is attained.

When the treatment cycle is completed in the final position, an acoustic signal of descending tone is emitted to indicate that the device has switched off.

The device also switches off automatically during the intermediate stages if the button has not been pressed for more than ten minutes.

Pressing the control knob at length results in early switch-off of the device.

The treatment cycle lasts two minutes for each position, for a total of nine positions.

The present invention provides uniform distribution of the light radiation over the scalp by virtue of the adaptable structure of the apparatus, and of the controlled movement.

The operation is simple, with minimal or no intervention by the user, who is able to apply the treatment substantially with the hands free, and without fatigue.

There is maximum comfort during use due to the lightness of the apparatus, which is worn as a pair of spectacles and does not impede either the view or blood circulation through the veins relating to the top of the head.

There is maximum safety as the structure prevents direct irradiation of the eyes.

The apparatus provides maximum resistance to impact because of the extreme elasticity of the structure, as required to allow adaptation to the various head dimensions.

It is also easy to transport because of its lightness, its compactness and the self-sufficiency of the rechargeable batteries.

The materials used for the apparatus 10, and the dimensions, can be chosen at will according to requirements and to the state of the art.

The device for irradiating the scalp with light sources conceived in this manner is susceptible to numerous modifications and variants, all falling within the scope of the inventive concept; moreover all details can be replaced by technically equivalent elements.

## Claims

1. A device for irradiating the scalp with light sources, comprising: a crown (12) open on the rear, to be located on the head; two horizontally positioned first forks (15), closed at their ends, mounted above the side of said crown (12); two articulated joints (16) mounted on said first forks (15), one per side; two second forks (18), also closed at their ends, inserted into said two articulated joints (16); said two articulated joints (16) enabling said two first forks (15) to rotate relative to said two second forks (18); said second forks (18) being connected to a movable structure (20) said structure (20) comprising a plurality of light emitters (21); **characterised in that** said two articulated joints (16) are free to slide, under friction, along said two first forks (15) such as to move said movable structure (20) forwards or rearwards relative to the head; said two second forks (18) are free to slide, under friction, through said two articulated joints (16) such as to move said movable structure (20) towards or away from the head.

2. A device as claimed in claim 1, **characterised in that** said movable structure (20) incorporates said light sources (21) in its interior.

3. A device as claimed in claim 1, **characterised in that** said movable structure (20) is positionable both horizontally and vertically.

4. A device as claimed in claim 1, **characterised in that** said light sources (21) are adapted to emit light at wavelengths comprising all the wavelengths of the visible including the infrared and ultraviolet spectrum portions.

5. A device as claimed in claim 1, **characterised in that** said light sources (21) are adapted to emit focused light or non-focused light.

6. A device as claimed in claim 1, **characterised in that** the emission modality of said light sources (21) can be continuous or pulsed.

## Patentansprüche

1. Vorrichtung zum Bestrahlen der Kopfhaut mit Lichtquellen, die aufweist: eine an der Rückseite offene Krone (12), um am Kopf positioniert zu werden; zwei horizontal angeordnete erste Gabeln (15), die an ihren Enden geschlossen und über der Seite der Krone (12) montiert sind; zwei gelenkige Verbindungen (16), die an den ersten Gabeln (15) montiert sind, und zwar eine pro Seite; zwei zweite Gabeln (18), die ebenfalls an ihren Enden geschlossen und in die beiden gelenkigen Verbindungen (16) eingesetzt sind; wobei die beiden gelenkigen Verbindungen (16) es den beiden ersten Gabeln (15) ermöglichen, sich relativ zu den beiden zweiten Gabeln (18) zu drehen; wobei die zweiten Gabeln (18) mit einer bewegbaren Struktur (20) verbunden sind; wobei die Struktur (20) eine Mehrzahl von Licht-Emittern (21) aufweist; **dadurch gekennzeichnet, dass** die beiden gelenkigen Verbindungen (16) entlang der beiden ersten Gabeln (15) unter Reibung frei gleiten können, um die bewegbare Struktur (20) relativ zum Kopf vorwärts und rückwärts bewegen zu können; die beiden zweiten Gabeln (18) durch die beiden gelenkigen Verbindungen (16) unter Reibung frei gleiten können, um die bewegbare Struktur (20) in Richtung auf den Kopf und weg von dem Kopf bewegen zu können.

2. Vorrichtung nach Anspruch 1, **dadurch gekennzeichnet, dass** die bewegbare Struktur (20) die Lichtquellen (21) in ihrem Inneren enthält.

3. Vorrichtung nach Anspruch 1, **dadurch gekennzeichnet, dass** die bewegbare Struktur (20) sowohl horizontal als auch vertikal positionierbar ist.

4. Vorrichtung nach Anspruch 1, **dadurch gekennzeichnet, dass** die Lichtquellen (21) ausgestaltet sind, um Licht mit Wellenlängen zu emittieren, die alle Wellenlängen des sichtbaren Lichts umfassen, einschließlich infrarote und ultraviolette Spektralbereiche.

5. Vorrichtung nach Anspruch 1, **dadurch gekennzeichnet, dass** die Lichtquellen (21) ausgestaltet sind, um fokussiertes Licht oder nicht-fokussiertes Licht zu emittieren.

6. Vorrichtung nach Anspruch 1, **dadurch gekennzeichnet, dass** der Emissionsmodus der Lichtquellen (21) kontinuierlich oder pulsierend sein kann.

## Revendications

1. Dispositif permettant d'irradier le cuir chevelu au moyen de sources lumineuses, comprenant:
une couronne (12) ouverte sur l'arrière, de manière à être placée sur la tête ;
deux premières fourches (15) positionnées horizontalement, fermées au niveau de leur extrémité, montées au-dessus du côté de ladite couronne (12) ; deux jointures articulées (16) montées sur lesdites premières fourches (15), une par côté ; deux secondes fourches (18), également fermées au niveau de leurs extrémités, insérées dans lesdites deux jointures articulées (16) ; lesdites deux jointures articulées (16) permettant auxdites deux premières fourches (15) de pivoter par rapport auxdites deux secondes fourches (18) ; lesdites secondes fourches (18) étant connectées à une structure mobile (20), ladite structure mobile (20) comprenant une pluralité d'émetteurs lumineux (21) ; **caractérisé en ce que** lesdites deux jointures articulées (16) sont libres de coulisser, sous l'action d'une friction, le long desdites deux premières fourches (15) de manière à déplacer ladite structure mobile (20) vers l'avant ou vers l'arrière par rapport à la tête ; lesdites deux secondes fourches (18) sont libre de coulisser, sous l'action d'une friction, au travers desdites deux jointures articulées (16) de manière à déplacer ladite structure mobile (20) au dessus ou à distance de la tête.

2. Dispositif selon la revendication 1, **caractérisé en ce que** ladite structure mobile (20) incorpore lesdites sources lumineuses (21) en son sein.

3. Dispositif selon la revendication 1, **caractérisé en ce que** ladite structure mobile (20) est positionnable à la fois horizontalement et verticalement.

4. Dispositif selon la revendication 1, **caractérisé en ce que** lesdites sources lumineuses (21) sont configurées pour émettre de la lumière sur des longueurs d'onde comprenant toutes les longueurs d'onde du spectre visible y compris les parties du spectre correspondant aux ultraviolets et aux infrarouges.

5. Dispositif selon la revendication 1, **caractérisé en ce que** lesdites sources lumineuses (21) sont configurées pour émettre de la lumière dirigée ou de la lumière diffuse.

6. Dispositif selon la revendication 1, **caractérisé en ce que** la modalité démission desdites sources de lumières (21) peut être continue ou pulsée.
